(19) Europäisches Patentamt
European Patent Office
Office européen des brevets

(11) EP 0 671 161 B1

(12) **EUROPÄISCHE PATENTSCHRIFT**

(45) Veröffentlichungstag und Bekanntmachung des Hinweises auf die Patenterteilung:
**22.10.2003 Patentblatt 2003/43**

(51) Int Cl.[7]: **A61K 7/48**, C07D 239/06

(21) Anmeldenummer: **94119407.8**

(22) Anmeldetag: **08.12.1994**

(54) **Ectoin und Ectoinderivate als Feuchtigkeitsspender in Kosmetikprodukten**

Ectoine and ectoine derivatives as moisturizing agents in cosmetic preparation

Ectoine et dérivés de l'ectoine comme agents hydratants dans des compositions cosmétiques

(84) Benannte Vertragsstaaten:
**AT BE CH DE DK ES FR GB GR IE IT LI LU NL PT SE**

(30) Priorität: **14.12.1993 DE 4342560**

(43) Veröffentlichungstag der Anmeldung:
**13.09.1995 Patentblatt 1995/37**

(73) Patentinhaber: **MERCK PATENT GmbH**
**64293 Darmstadt (DE)**

(72) Erfinder:
- **Motitschke, Lothar, Prof. Dr.**
  **D-40723 Hilden (DE)**
- **Driller, Hansjürgen, Dr.**
  **D-40789 Monheim (DE)**
- **Galinski, Erwin, Dr.**
  **D-53127 Bonn (DE)**

(56) Entgegenhaltungen:
**EP-A- 0 088 542**
**EP-A- 0 553 884**
**WO-A-94/15923**
**DE-A- 2 132 079**
**DE-A- 2 154 948**

- **PATENT ABSTRACTS OF JAPAN vol. 15, no. 154 (C-825) & JP-A-03 031 265 (TAKEDA CHEM. IND. LTD.)**
- **PATENT ABSTRACTS OF JAPAN vol. 15, no. 259 (C-846) & JP-A-03 086 867 (TAKEDA CHEM. IND. LTD.)**
- **DATABASE WPI Derwent Publications Ltd., London, GB; AN 90-[161349] & JP-A-2 104 577 (KOEI CHEM. IND. KK)**
- **EUR. J. BIOCHEM., Bd.149, Nr.1, 1985 Seiten 135 - 139 E.A. GALINSKI '1,4,5,6-Tetrahydro-2-methyl -4-pyrimidinecarboxylic acid'**
- **STN INTERNATIONAL, KARLSRUHE. & FRAGRANCE J. (1991), 19(2), 70-7**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 671 161 B1

Printed by Jouve, 75001 PARIS (FR)

## Beschreibung

Es ist bekannt, daß (S)-1,4,5,6-Tetrahydro-2-methyl-4-pyrimidincarbonsäure (Ectoin) in extrem halophilen Mikroorganismen vorkommt und bei der Osmoregulation dieser Organismen eine Rolle spielt (E.A. Galinski et al., Eur. J. Biochem., 149 (1985) Seiten 135-139).

Aus E.A. Galinski, H.-P. Pfeiffer, H.G. Trüper, "1,4,5,6-Tetrahydro-2-methyl-4-pyrimidinecarboxylic acid" Eur. J. Biochem, 149 (1985) 135-139 ist Ectoin als Substanz bekannt, die von halophilen Mikroorganismen gebildet wird. Die Extraktion der Substanz aus Ectothiorhodospira halochloris und die Strukturaufklärung sind beschrieben. Auch der Trivialname "Ectoin" für 1,4,5,6-Tetrahydro-2-methyl-4-pyrimidincarboxylsäure wird in dieser Publikation geprägt. Weiter wird in der Publikation vermutet, dass Ectoin an der Osmoregulation dieser halophilen Mikroorganismen beteiligt ist

Eine chemische Synthese für Tetrahydropyrimidin-derivate, wie Ectoin, wird in JP-A-03-31265 beschrieben. Dabei wird 2,4-Diaminobuttersäure bzw. ein Ester davon mit einer Verbindung CH3-X, wobei X beispielsweise -CN oder $-C(OR)_3$ steht, umgesetzt.

Aus der Europäischen Patentanmeldung EP-A-0 553 884 sind pharmazeutische Zusammensetzungen bekannt, die als aktiven Inhaltstoff Ectoin und/oder Hydroxyectoin zusammen mit einem geeigneten Träger enthärten. Diese Zusammensetzungen eignen sich zum Schutz der DNA von tumorfreiem Gewebe gegen Schädigung durch DNA-bindende Wirkstoffe, Carcinogene, Mutagene oder UV-Strahlung. Diese Zubereitungen werden dabei vorzugsweise intravenös verabreicht.

Aus der nicht-vorveröffentlichten Deutschen Patentanmeldung DE-A-42 44 560 ist ein Verfahren zur Gewinnung von Ectoin und Hydroxyectoin aus halophilen Mikroorganismen bekannt. Weiter ist aus dieser Patentanmeldung bekannt, dass Ectoin und Hydroxyectoin zur Stabilisierung von Enzymen gegen denaturierende Einwirkungen und insbesondere Hydroxyectoin zur Kryoprotektion von biologischen Wirkstoffen, Zellen, Blutkonserven in der Medizintechnik, der pharmazeutischen und/oder kosmetischen Industrie geeignet ist. Auch die Verwendung von Hydroxyectoin zu Herstellung eines Arzneimittels zur Hautbehandlung ist aus diesem Dokument bekannt.

Eine Hauptaufgabe kosmetischer Produkte ist die Erhaltung oder Wiederherstellung des Normalzustandes der Haut. Neben anderen Kriterien spielt die Stabilisierung des Feuchtigkeitsgehaltes der Haut eine wesentliche Rolle. Die Haut ist unter normalen Verhältnissen selbständig in der Lage, ihren Feuchtigkeitsgehalt zu regulieren. Durch Veränderung der äußeren Einflüsse, wie z.B. bei kalter trockener Atmosphäre, wird sehr schnell der Zustand einer ausgetrockneten Hautoberfläche erreicht. Die Oberfläche der Haut wird schuppig und neigt zu kleinen Einrissen. Die Haut wird hochempfindlich gegen chemische und physikalische Einflüsse. Dieses Hautbild manifestiert sich bei Atopikern altersunabhängig, bei gesunden Menschen ist eine Zunahme des trockenen Hautzustandes mit zunehmendem Alter zu beobachten (B. Idson, Cosmetics & Toiletries, 107 (1992), Seiten 69-78). Durch Verwendung von entsprechenden feuchtigkeitsspendenden Präparationen kann diesem Hautzustand sowohl vorgebeugt als auch entgegengewirkt werden.

Die zu diesem Zweck in feuchtigkeitsspendenden Präparationen verwendeten Inhaltsstoffe werden durch zwei unterschiedliche Wirkprinzipien unterschieden.

Die okklusiven Substanzen (z.B. Paraffinöle) bilden eine wasserdampfundurchlässige Barriere auf der Hautoberfläche und verhindern hierdurch den transepidermalen Wasserverlust (TEWL) der Haut. Intradermale Substanzen wie Glyzerin binden dagegen das Wasser in der Haut.

Es wurde nun gefunden, daß kosmetische Zubereitungen, enthaltend mindestens eine Verbindung der Formel Ia oder Ib, die Hydratation der Humanhaut erhöhen und stabilisieren.

Die Erfindung betrifft die Verwendung von mindestens einer Verbindung der Formel Ia oder Ib

(Ia) 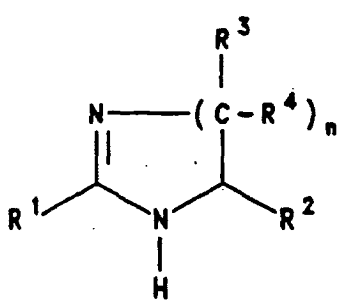

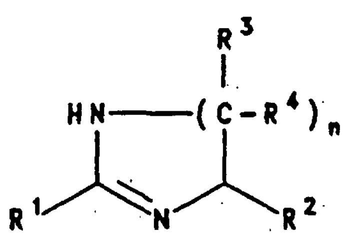 (Ib)

und/oder eines physiologisch verträglichen Salzes der Verbindung der Formel Ia oder Ib und/oder einer stereoisomeren Form der Verbindung der Formel Ia oder Ib zur Herstellung von kosmetischen Zubereitungen; dabei hat $R^1$ die nach-

stehende Bedeutung:

a) Wasserstoffatom oder
b) ($C_1$-$C_4$)-Alkyl,

dabei hat $R^2$ die nachstehende Bedeutung:

a) Wasserstoffatom,
b) -COOH,
c)

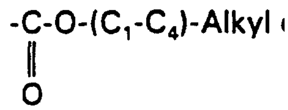

oder
d)

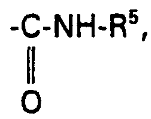

wobei $R^5$ für

1) Wasserstoffatom,
2) ($C_1$-$C_4$)-Alkyl,
3) Aminosäurerest,
4) Dipeptidrest oder
5) Tripeptidrest steht,

dabei haben $R^3$ und $R^4$ unabhängig voneinander die nachstehende Bedeutung:

a) Wasserstoffatom oder
b) -OH und

n ist die Zahl 1, 2 oder 3.

Die Verbindungen der Formel Ia oder Ib können in den kosmetischen Zubereitungen als optische Isomere, Diastereomere, Racemate, Zwitterionen, Kationen oder als Gemisch derselben vorliegen.

Bevorzugt ist die Verwendung von mindestens einer Verbindung der Formel Ia oder Ib, dabei hat $R^1$ die nachstehende Bedeutung:

a) Wasserstoffatom oder
b) Methyl,

$R^2$ hat die nachstehende Bedeutung:

a) Wasserstoffatom oder
b) -COOH,

$R^3$ und $R^4$ haben unabhängig voneinander die nachstehende Bedeutung:

a) Wasserstoffatom oder
b) -OH, und

n ist die Zahl 2.

Die Erfindung betrifft ferner kosmetische Zubereitungen in Form von Emulsionen, enthaltend (S)-1,4,5,6-Tetrahydro-2-methyl-4-pyrimidincarbonsäure und/oder (S,S)-1,4,5,6-Tetrahydro-5-hydroxy-2-methyl-4-pyrimidincarbonsäure.

Die Erfindung betrifft weiterhin Verfahren zur Herstellung der Verbindung der Formel Ia oder Ib, wobei man

A) eine Verbindung der Formel II

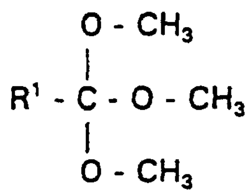

(II)

wobei $R^1$ die in Formel Ia genannte Bedeutung hat,
mit einer Verbindung der Formel III

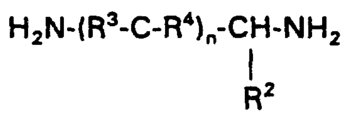

(III),

in Gegenwart von einem Alkanol zu einer Verbindung der Formel Ia oder Ib umsetzt,
wobei $R^3$, $R^4$ und n die in Formel Ia genannte Bedeutung haben und $R^2$ Wasserstoffatom oder -COOH ist, oder

B) eine Verbindung der Formel Ia oder Ib, wobei $R^2$ -COOH ist, mit einem Alkylhalogenid in die entsprechenden Carbonsäureester der Verbindung der Formel Ia oder Ib umsetzt, oder

C) eine Verbindung der Formel Ia oder Ib, wobei $R^2$ ein aktivierter Carbonsäureester ist, mit einem Amin der Formel $NH_2$-$R^5$ in das entsprechende Carbonsäureamid der Verbindung der Formel Ia oder Ib umsetzt.

Unter dem Begriff "Aminosäure" werden die stereoisomeren Formen, z.B. D-und L-Formen, folgender Verbindungen verstanden:
Asparagin, Arginin, Asparaginsäure, Glutamin, Glutaminsäure, β-Alanin, γ-Aminobutyrat, Nε-Acetyllysin, Nδ-Acetylornithin, Nγ-Acetyldiaminobutyrat, Nα-Acetyldiaminobutyrat, Histidin, Isoleucin, Leucin, Methionin, Phenylalanin, Serin, Threonin und Tyrosin. L-Aminosäuren sind bevorzugt. Aminosäurereste leiten sich von den entsprechenden Aminosäuren ab.

Folgende Aminosäurereste sind bevorzugt:
Gly, Ala, Ser, Thr, Val, β-Ala, γ-Aminobutyrat, Asp, Glu, Asn, Gln, Nε-Acetyllysin, Nδ-Acetylornithin, Nγ-Acetyldiaminobutyrat, Nα-Acetyldiaminobutyrat.

Die Kurzschreibweise der Aminosäuren erfolgte nach der allgemein üblichen Schreibweise. Die Di- oder Tripeptidreste sind ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Geeignete physiologisch verträgliche Salze der Verbindung der Formel Ia oder Ib sind beispielsweise Alkali-, Erdalkali- oder Ammoniumsalze wie Na-, K-, Mg-, Ca-, Triethylamin- oder Tris-(2-hydroxy-ethyl)-aminsalze. Weitere physiologisch verträgliche Salze der Verbindung der Formel Ia oder Ib ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure. Verbindungen der Formel Ia oder Ib, in denen basische und saure Gruppen wie Carboxyl- oder Aminogruppen in gleicher Zahl vorliegen, bilden innere Salze.

Ein Verfahren zur Herstellung von Di- oder Tripeptiden oder deren Salzen wird offenbart, das dadurch gekennzeichnet ist, daß man

a) ein Segment mit C-terminaler freier Carboxylgruppe oder dessen aktiviertes Derivat mit einem entsprechenden Segment mit N-terminaler freier Aminogruppe umsetzt oder
b) das Di- oder Tripeptid stufenweise aufbaut, und

in der nach a) oder b) erhaltenen Verbindung gegebenenfalls eine oder mehrere zum Schutz anderer Funktionen temporär eingeführte Schutzgruppen abspaltet und die so erhaltenen Verbindungen gegebenenfalls in ihr physiologisch verträgliches Salz überführt.

Die Di- oder Tripeptide werden nach den allgemeinen Methoden der Peptidchemie stufenweise vom C-terminalen Ende her oder durch Kupplung von Segmenten hergestellt (Houben-Weyl, Methoden der Organischen Chemie, Band 15/1,2). Die Peptidkupplungen können z.B. nach der Methode der gemischten Anhydride über Aktivreste oder Azide oder nach der Carbodiimid-Methode, insbesondere unter Zusatz reaktionsbeschleunigender und racemisierungsverhindernder Substanzen wie 1-Hydroxybenzotriazol, N-Hydroxysuccinimid, 3-Hydroxy-4-oxo-3.4-dihydro-1.2.3-benzotriazin, N-Hydroxy-5-norbornen-2.3-dicarboximid, ferner unter Verwendung aktiver Derivate des 1-Hydroxybenzotriazols oder Anhydriden von Phosphor-, Phosphonund Phosphinsäuren, bei einer Reaktionstemperatur von -10°C bis zum Siedepunkt des Lösungsmittels, vorzugsweise von -5°C bis 40°C, durchgeführt werden.

Geeignete Lösungsmittel dafür sind Dimethylformamid, Dimethylacetamid, N-Methylpyrrolidon oder Dimethylsulfoxid. Sofern die Löslichkeit der Komponenten es erlaubt, können auch Lösungsmittel wie Methylenchlorid, Chloroform oder Tetrahydrofuran eingesetzt werden.

Falls zur Verhinderung von Nebenreaktionen oder für die Synthese spezieller Peptide erforderlich, sind die funktionellen Gruppen in der Seitenkette von Aminosäuren durch geeignete Schutzgruppen zusätzlich geschützt, wobei in erster Linie Arg(Tos), Arg(Mts), Arg(Mtr), Arg(PMV), Asp(OBzl), Asp(OBut), Cys(4-MeBzl), Cys(Asm), Cys(SBut), Glu(Obzl), Glu(Obut), His(Tos), His(Fmoc), His(Dnp), His(Trt), Lys(Cl-Z), Lys(Boc), Met(O), Ser(Bzl), Ser(But), Thr (Bzl), Thr(But), Trp(Mts), Trp(CHO), Tyr(Br-Z), Tyr(Bzl) oder Tyr(But) eingesetzt wird.

Als Aminoschutzgruppen werden bevorzugt der durch katalytische Hydrierung abspaltbare Benzyloxycarbonyl-(Z)Rest, der durch schwache Säuren abspaltbare 2-(3,5-Dimethyloxyphenyl)propyl(2)oxycarbonyl(Ddz-) oder Trityl-(Trt)-Rest und der durch sekundäre Amine abspaltbare 9-Fluorenylmethyloxycarbonyl-(Fmoc)-Rest herangezogen.

Bei der Verfahrensvariante A) geht man am besten so vor, daß man die Verbindung der Formel II in äquimolarer Menge oder in einem bis zu dreifachen Überschuß in einem inerten Lösungsmittel wie Methanol, Ethanol oder Isopropanol mit einer Verbindung der Formel III (in der Dihydrochlorid-Form) unter ständigem Rühren zu einer Verbindung der Formel la oder Ib umsetzt. Die Reaktionstemperaturen liegen bei 30 bis 90°C oder bei 30°C bis zum Siedepunkt des Lösungsmittels, vorzugsweise bei 40 bis 70°C, insbesondere um 55°C.

Die Reaktionszeiten betragen von 15 min bis 48 Stunden, bevorzugt von 30 min bis 3 Stunden, insbesondere bevorzugt von 1 bis 2 Stunden. Die Beendigung der Reaktion kann beispielsweise mittels HPLC bestimmt werden.

Zur Isolierung und Reinigung der Reaktionsprodukte der Formel la oder Ib kann man das Lösungsmittel abdestillieren, die getrocknete Reaktionsmischung in demineralisiertem Wasser mit Säuren oder Laugen neutralisieren und zwitterionische Produkte auf einer lonenverzögerungssäule (beispielsweise vom Typ BIORAD® AG11A8) mit demineralisiertem Wasser als Laufmittel reinigen. Kationische Produkte werden zur Reinigung an einen Kationenaustauscher in der $H^+$-Form gebunden und beispielsweise mit einem Perchlorsäure-Gradienten eluiert.

Die erhaltene Verbindung der Formel Ia oder Ib, wobei $R^2$ -COOH ist, wird in den entsprechenden Carbonsäureester überführt (Verfahrensvariante B), indem man beispielsweise 1 g des Hydrochlorids der Verbindung der Formel la oder Ib in 20 ml 0,2 N HCl in Methanol löst, 2 Stunden unter Rückfluß erhitzt und anschließend unter vermindertem Druck eindampft. Die Veresterung wird wie im Falle von Nitrophenylestern durch Zusatz von äquimolaren Mengen an Dicyclohexylcarbodiimid als wasserbindendem Agens beschleunigt.

Die entsprechenden Carbonsäureamide der Formel la oder Ib (Verfahrensvariante C) kann man erhalten, indem man 1 g eines aktivierten Carbonsäureesters (beispielsweise p-Nitrophenylester) der Verbindung der Formel la oder Ib in 250 ml Dichlormethan löst und mit gasförmigen $NH_3$ oder mit der entsprechenden Aminosäure oder einem Di- oder Tripeptid (unter Zusatz von Triäthylamin) zur Reaktion bringt. Die Reaktion kann anhand des sich bildenden p-Nitrophenolats (Gelbfärbung) verfolgt werden.

Die Ausgangsverbindungen der Verfahrensvariante A) sind bekannt oder können käuflich erworben werden. (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure können auch mikrobiologisch gewonnen werden (Severin et al. J. Gen. Microb. (1992), 138, Seiten 1629-1638).

Die Herstellung der kosmetischen Zubereitung erfolgt, indem mindestens eine Verbindung der Formel la oder Ib und/oder ein physiologisch verträgliches Salz der Verbindung der Formel la oder Ib gegebenenfalls mit Hilfs- und/ oder Trägerstoffen in eine geeignete Zubereitungsform gebracht wird. Die Hilfs- und Trägerstoffe stammen aus der Gruppe der Trägermittel, Konservierungsstoffe und anderer üblicher Hilfsstoffe.

Die kosmetischen Zubereitungen auf der Grundlage der Verbindung der Formel Ia oder Ib werden äußerlich angewendet.

Als Anwendungsform seien z.B. genannt: Lösungen, Suspensionen, Emulsionen, Pasten, Salben, Gele, Cremes, Lotionen, Puder, Seifen, tensidhaltige Reinigungspräparate, Öle und Sprays. Zusätzlich zu der Verbindung der Formel Ia oder Ib werden der Zubereitung beliebige übliche Trägerstoffe, Hilfsstoffe und gegebenenfalls weitere Wirkstoffe zugesetzt.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können neben der Verbindung der Formel Ia oder Ib die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben der Verbindung der Formel Ia oder Ib die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können neben der Verbindung der Formel Ia oder Ib die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben der Verbindung der Formel Ia oder Ib die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können neben der Verbindung der Formel Ia oder Ib die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glyzerin, Zucker oder Gemische dieser Stoffe enthalten.

Tensidhaltige Reinigungsprodukte können neben der Verbindung der Formel Ia oder Ib die üblichen Trägerstoffe wie Salze von Fettalkoholsulfaten, Fettalkoholethersulfaten, Sulfobernsteinsäurehalbestern, Fettsäureeiweißhydrolysaten, Isethionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben der Verbindung der Formel Ia oder Ib die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Rouge, Puder-, Emulsions und Wachs-Make up sowie Sonnenschutz- und After Sun-Präparate.

Die wirksame Konzentration der Verbindung der Formel Ia oder Ib in der erfindungsgemäßen kosmetischen Zubereitung beträgt von 0,1 bis 10 Gew.-%, vorzugsweise von 0,1 bis 3 Gew.-%.

Die Erfindung betrifft ferner die Verwendung der Verbindung der Formel Ia oder Ib zur Herstellung von kosmetischen Zubereitungen zur Pflege und Prophylaxe von trockener und/oder gereizter Haut und von trockener schuppiger Kopfhaut, insbesondere zur Erhöhung und/oder Stabilisierung des Feuchtigkeitsgehaltes der Haut.

Beispiel 1

Herstellung von (S)-1,4,5,6-Tetrahydro-2-methyl-4-pyrimidincarbonsäure

60 ml Orthoessigsäuretrimethylester werden in 400 ml Methanol gelöst und unter ständigem Rühren bei 55°C mit 30 g L-Diaminobuttersäure x 2 HCl umgesetzt. Nach 2 Stunden Reaktionszeit wird das Lösungsmittel unter vermindertem Druck eingedampft, das verbleibende Rohprodukt wird in 50 ml demineralisiertem Wasser gelöst und mit NaOH neutralisiert. Anschließend wird die Verbindung auf einer Ionenverzögerungssäule vom Typ BIORAD® AG11A8 (Bio-Rad Laboratories GmbH, München, Deutschland) mit demineralisiertem Wasser als Laufmittel gereinigt, getrocknet und aus Methanol umkristallisiert. Die erhaltene Verbindung kann mit einer Hochdruckflüssigchromatographie (HPLC) identifiziert werden (beispielsweise Firma E. Merck, Darmstadt, Deutschland, LiChroCART® 125-$NH_2$ 5μ, Laufmittel 80 % Acetonitril) und ist durch die folgenden $^{13}C$-NMR-Daten charakterisiert:
176.8, 161.2, 53.8, 37.9, 22.0, 18.7 ppm (relativ zu TMS); (Galinski E.A., Pfeiffer H.P., Trüper H.G. (1985) Eur. J. Biochem. 149, 135-139).

Beispiel 2

Kosmetische Wirksamkeit

A) Wasseraufnahmevermögen

Die Wasseraufnahmekapazität der Verbindung nach Beispiel 1 wird bei einer relativen Luftfeuchtigkeit von 65 % bestimmt. Als Kontrollsubstanzen werden jeweils Glyzerin und Propylenglycol verwendet. Jeweils 1 g der wasserfreien Substanz wird bis zur Gewichtskonstanz in einer geschlossenen Kammer aufbewahrt. Die Wasseraufnahmekapazität ergibt sich aus:

$$\text{Wasseraufnahme in \%} = \frac{\text{Gewicht der aufgenommenen Wassermenge}}{\text{Gewicht der getrockneten Substanz}} \times 100$$

Im Vergleich mit Glyzerin oder Propylenglycol (60 bzw. 45 %) besitzt Ectoin mit 25 % Wasseraufnahme ein weniger ausgeprägtes hygroskopisches Verhalten. Hinsichtlich der Verwendung als Feuchthaltesubstanz kann jedoch der bei zu hohen Glyzerinkonzentrationen bekannte austrocknende Effekt ausgeschlossen werden.

B) Hydratation der Humanhaut

Die Feuchtigkeitsmessung erfolgt auf kapazitiven Weg mit dem Corneometer® CM 820 (Courage & Khazaka Electronic GmbH). Dabei macht man sich die relativ hohe Dielektrizitätskonstante von Wasser zunutze. Die Stirnfläche des Meßfühlers enthält den Meßkondensator. Wird der Meßkopf auf die Haut gedrückt, gelangt die Hornschicht in den Streubereich des Kondensatorfeldes. Je nach Wassergehalt kommt es zu unterschiedlichen Kapazitätsänderungen. Durch die kurze Meßzeit sind Fehler durch Hautdeformationen oder Verdampfungsstau ausgeschlossen.

7 Probanden werden 14 Tage einmal täglich mit einer Emulsion, enthaltend (S)-1,4,5,6-Tetrahydro-2-methyl-4-pyrimidincarbonsäure (Verbindung gemäß Beispiel 1, Ectoin), am Unterarm behandelt. Zur Kontrolle wird die entsprechende Placebo-Emulsion am anderen Unterarm appliziert. Die mit dem Corneometer ermittelten Meßwerte zeigen, daß die Humanhaut, die mit der erfindungsgemäßen Emulsion behandelt wurde, eine etwa 10 % höhere Hydratation im direkten Vergleich mit der Placebo-Emulsion aufweist. Dies ist eine deutlich verbesserte und signifikant höhere Hydratation der Humanhaut als die durch die Placebo-Emulsion erreichte Hydratation.

Tabelle 1 zeigt die Zusammensetzung der Emulsionen.

Tabelle 1:

| Placebo Emulsion | |
|---|---|
| A K-Cetyl-Phosphate | 3,0 % |
| A Glyceryl Stearate | 4,0 % |
| A Methoxy PEG-17/Dodecyl Glycol Copolymer | 1,0 % |
| A Glyceryl Laurate | 2,0 % |
| A Ceteraryl Alcohol | 3.0 % |
| A Steareth-21 | 0,3 % |
| A Polymethylmethacrylate | 1,0 % |
| A Isocetyl Alcohol | 6,0 % |
| A Macadamia Nut Oil | 2,0 % |
| A Dimethicone | 2,0 % |
| A Caprylic/Capric Triglyceride | 4,0 % |
| A Perfluoropolymethylisopropyl Ether | 0,5 % |
| A Butylhydroxytoluol | 0,02 % |
| | |
| B gereinigtes Wasser | 61,23 % |

Tabelle 1: (fortgesetzt)

| | |
|---|---|
| Placebo Emulsion | |
| B Na-Carbomer | 0,25 % |
| B Sorbeth-30 | 3.0 % |
| B Glycerin | 2.0 % |
| B Methyl Gluceth-20 | 0,5 % |
| B Ethylendiamin-N,N,N',N'-Tetraessigsäure-Tetranatriumsalz | 0.05 % |
| B Panthenol | 2,0 % |
| B Konservierungsmittel | 1,9 % |
| B Magnesium Aluminium Silicate | 0,15 % |
| | |
| C Parfüm | 0,1 % |
| (S)-1,4,5,6-Tetrahydro-2-methyl-4-pyrimidincarbonsäure (Ectoin-Emulsion) | |
| A K-Cetyl-Phosphate | **3,0 %** |
| A Glyceryl Stearate | 4,0 % |
| A Methoxy PEG-17/Dodecyl Glycol Copolymer | 1,0 % |
| A Glyceryl Laurate | 2,0 % |
| A Ceteraryl Alcohol | 3,0 % |
| A Steareth-21 | 0,3 % |
| A Polymethylmethacrylate | 1,0 % |
| A Isocetyl Alcohol | 6,0 % |
| A Macadamia Nut Oil | 2,0 % |
| A Dimethicone | 2,0 % |
| A Caprylic/Capric Triglyceride | 4,0 % |
| A Perfluoropolymethylisopropyl Ether | 0,5 % |
| A Butylhydroxytoluol | 0,02 % |
| | |
| B gereinigtes Wasser | 60,23 % |
| B Na-Carbomer | 0,25 % |
| B Sorbeth-30 | 3,0 % |
| B Glycerin | 2,0 % |
| B Methyl Gluceth-20 | 0,5 % |
| B Ethylendiamin-N,N,N',N'-tetraessigsäure-tetranatriumsalz | 0,05 % |
| B Panthenol | 2,0 % |
| B Konservierungsmittel | 1,9 % |
| B Magnesium Aluminium Silicate | 0,15 % |
| | |
| C Parfüm | 0,1 % |
| | |
| D (S)-1,4,5,6-Tetrahydro-2-methyl-4-pyrimidincarbonsäure | 1,0 % |

**Patentansprüche**

1. Kosmetische Verwendung von mindestens einer Verbindung der Formel Ia oder Ib

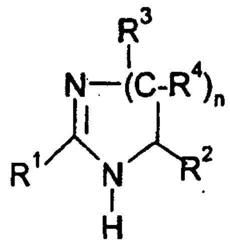

Ia

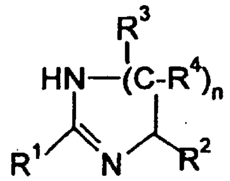

Ib

und/oder eines physiologisch verträglichen Salzes der Verbindung der Formel Ia oder Ib und/oder einer stereoisomeren Form der Verbindung der Formel Ia oder Ib zur Erhöhung und/oder Stabilisierung des Feuchtigkeitsgehaltes der Haut,
dabei hat $R^1$ die nachstehende Bedeutung:

a) Wasserstoffatom oder
b) ($C_1$-$C_4$)-Alkyl,

dabei hat $R^2$ die nachstehende Bedeutung:

a) Wasserstoffatom,
b) -COOH,
c)

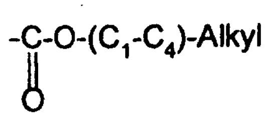

oder
d)

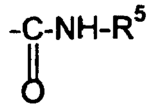

wobei $R^5$ für

1) Wasserstoffatom,
2) ($C_1$-$C_4$)-Alkyl,
3) Aminosäurerest,
4) Dipeptidrest oder
5) Tripepdidrest steht,

dabei haben $R^3$ und $R^4$ unabhängig voneinander die nachstehende Bedeutung:

a) Wasserstoffatom oder
b) -OH und

n ist die Zahl 1, 2 oder 3,

**2.** Verwendung gemäß Anspruch 1, **dadurch gekennzeichnet, daß** man eine Verbindung der Formel Ia oder Ib einsetzt;
dabei hat $R^1$ die nachstehende Bedeutung:

a) Wasserstoffatom oder
b) Methyl,

$R^2$ hat die nachstehende Bedeutung:

a) Wasserstoffatom oder
b) -COOH,

$R^3$ und $R^4$ haben unabhängig voneinander die nachstehende Bedeutung:

a) Wasserstoffatom oder
b) -OH, und

n ist die Zahl 2.

**3.** Verwendung gemäß Anspruch 1 oder 2, **dadurch gekennzeichnet, daß** man (S)-1,4,5,6-Tetrahydro-2-methyl-4-pyrimidincarbonsäure und/oder (S,S)-1,4,5,6-Tetrahydro-5-hydroxy-2-methyl-4-pyrimidincarbonsäure einsetzt.

**4.** Verwendung von der Verbindung der Formel Ia oder Ib nach einem oder mehreren der Ansprüche 1 bis 3, **dadurch gekennzeichnet, daß** man die kosmetische Zubereitung in Form einer Lösung, einer Suspension, einer Emulsion, einer Paste, einer Salbe, eines Gels, einer Creme, einer Lotion, eines Puders, einer Seife, eines tensidhaltigen Reinigungsproduktes, eines Öls, eines Lippenstifts, eines Lippenpflegestifts, einer Mascara, eines Eyeliners, von Rouge, eines Puder-, Emulsions oder Wachs-Make ups, eines Sonnenschutz- und After Sun-Präparats oder eines Sprays einsetzt.

**5.** Verwendung von der Verbindung der Formel Ia oder Ib, nach einem oder mehreren der Ansprüche 1 bis 4 zur Herstellung von kosmetischen Zubereitungen, zur Pflege und Prophylaxe.von gealteter, trockener und/oder gereizter Haut und von trockener, schuppiger Kopfhaut.

**6.** Kosmetische Zubereitung enthaltend mindestens eine Verbindung der Formel Ia oder Ib

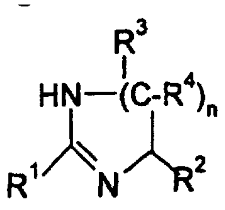

Ib

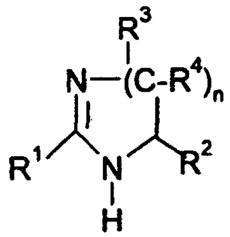

Ia

und/oder ein physiologisch verträgliches Salz der Verbindung der Formel Ia oder Ib und/oder eine stereoisomere Form der Verbindung der Formel Ia oder Ib
dabei hat $R^1$ die nachstehende Bedeutung:

a) Wasserstoffatom oder
b) $(C_1\text{-}C_4)$-Alkyl,

dabei hat $R^2$ die nachstehende Bedeutung:

a) Wasserstoffatom,

b) -COOH,

c)

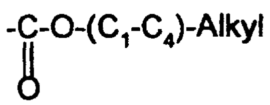

oder

d)

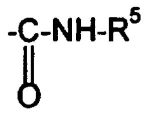

wobei $R^5$ für

1) Wasserstoffatom,
2) $(C_1\text{-}C_4)$-Alkyl,
3) Aminosäurerest,
4) Dipeptidrest oder
5) Tripeptidrest steht,

dabei haben $R^3$ und $R^4$ unabhängig voneinander die nachstehende Bedeutung:

a) Wasserstoffatom oder
b) -OH und

n ist die Zahl 1, 2 oder 3,
**dadurch gekennzeichnet, dass** es sich bei der Zubereitung um eine Emulsion handelt.

## Claims

1. Cosmetic use of at least one compound of the formula Ia or Ib

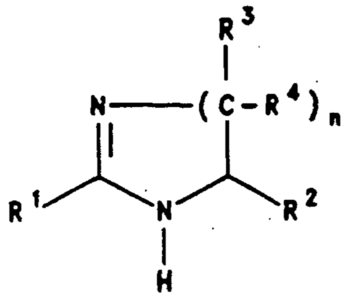

1a

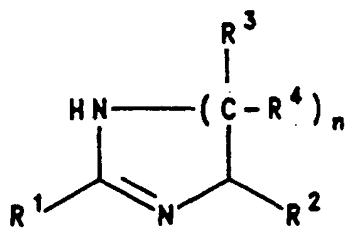

1b

and/or of a physiologically acceptable salt of the compound of the formula Ia or Ib and/or of a stereoisomeric form of the compound of the formula Ia or Ib for increasing and/or stabilizing the moisture content of the skin,
$R^1$ being defined as follows:

a) a hydrogen atom or
b) ($C_1$-$C_4$)-alkyl,

$R^2$ being defined as follows:

a) a hydrogen atom,
b) -COOH,
C)

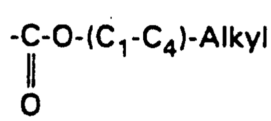

or

d) 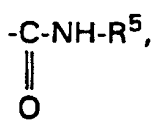

in which $R^5$ is

1) a hydrogen atom,
2) ($C_1$-$C_4$)-alkyl,
3) an amino acid radical,
4) a dipeptide radical or
5) a tripeptide radical,

$R^3$ and $R^4$ independently of one another being defined as follows:

a) a hydrogen atom or
b) -OH, and

n is the number 1, 2 or 3.

**2.** Use according to Claim 1, **characterized in that** a compound of the formula Ia or Ib is employed; $R^1$ being defined as follows:

a) a hydrogen atom or
b) methyl,

$R^2$ being defined as follows:

a) a hydrogen atom or
b) -COOH,

$R^3$ and $R^4$ independently of one another being defined as follows:

a) a hydrogen atom or
b) -OH, and

n is the number 2.

**3.** Use according to Claim 1 or 2, **characterized in that** (S)-1,4,5,6-tetrahydro-2-methyl-4-pyrimidine-carboxylic acid and/or (S,S)-1,4,5,6-tetrahydro-5-hydroxy-2-methyl-4-pyrimidinecarboxylic acid is employed.

**4.** Use of the compound of the formula Ia or Ib according to one or more of Claims 1 to 3, **characterized in that** the cosmetic product is employed in the form of a solution, a suspension, an emulsion, a paste, an ointment, a gel, a cream, a lotion, a powder, a soap, a surfactant-containing cleanser, an oil, a lipstick, a lipsalve stick, a mascara, an eyeliner, a blusher, a powder foundation, an emulsion foundation or a wax foundation, a suncare and after-sun preparation or a spray.

**5.** Use of the compound of the formula Ia or Ib according to one or more of Claims 1 to 4 for the preparation of cosmetic products for the care and prophylaxis of aged, dry and/or irritated skin and of dry, flaky scalp.

**6.** Cosmetic product comprising at least one compound of the formula.

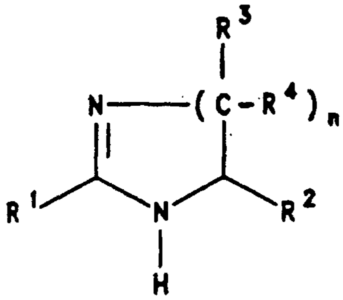

1a

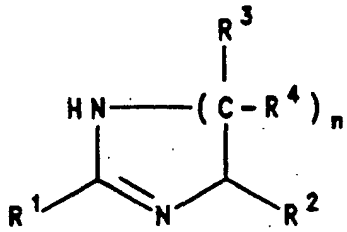

1b

and/or of a physiologically acceptable salt of the compound of the formula Ia or Ib and/or of a stereoisomeric form of the compound of the formula Ia or Ib;
$R^1$ being defined as follows:

a) a hydrogen atom or
b) $(C_1-C_4)$-alkyl,

$R^2$ being defined as follows:

a) a hydrogen atom,
b) -COOH,

c) 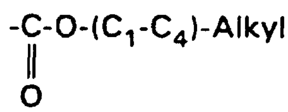

or
d)

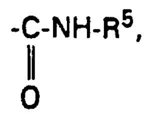

in which $R^5$ is

1) a hydrogen atom,
2) $(C_1-C_4)$-alkyl,
3) an amino acid radical,
4) a dipeptide radical or
5) a tripeptide radical,

$R^3$ and $R^4$ independently of one another being defined as follows:

a) a hydrogen atom or
b) -OH, and

n is the number 1, 2 or 3,
**characterized in that** the product is an emulsion.

## Revendications

**1.** Utilisation en cosmétologie d'au moins un composé de formule Ia ou Ib

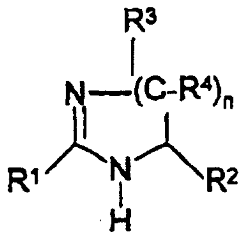

Ia

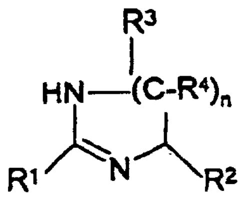

Ib

et/ou d'un sel physiologiquement compatible du composé de formule Ia ou Ib et/ou d'une forme stéréoisomère du composé de formule Ia ou Ib pour élever et/ou stabiliser la teneur en eau de la peau,

$R^1$ ayant la signification ci-après :

a) un atome d'hydrogène ou
b) un alkyle en $C_1$-$C_4$,

$R^2$ ayant la signification suivante :

a) un atome d'hydrogène,
b) -COOH,
c)

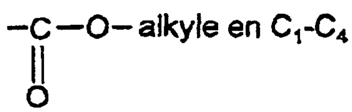

ou
d)

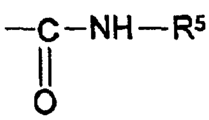

$R^5$ représentant

1) un atome d'hydrogène,
2) un alkyte en $C_1$-$C_4$,
3) un reste acide aminé,
4) un reste dipeptide, ou

5) un reste tripeptide,

$R^3$ et $R^4$ ayant, indépendamment l'un de l'autre, la signification ci-après :

a) un atome d'hydrogène ou
b) -OH et

n est égal à 1, 2 ou 3.

**2.** Utilisation selon la revendication 1, **caractérisée en ce que** l'on met en oeuvre un composé de formule Ia ou Ib, $R^1$ ayant la signification ci-après :

a) un atome d'hydrogène ou
b) le méthyle,

$R^2$ ayant la signification ci-après :

a) un atome d'hydrogène ou
b) -COOH,

$R^3$ et $R^4$ ayant, indépendamment l'un de l'autre, la signification ci-après :

a) un atome d'hydrogène ou
b) -OH et

n est égal à 2.

**3.** Utilisation selon la revendication 1 ou 2, **caractérisée en ce que** l'on met en oeuvre l'acide (S)-1,4,5,6-tétrahydro-2-méthyl-4-pyrimidine-carboxylique et/ou l'acide (S,S)-1,4,5,6-tétrahydro-5-hydroxy-2-méthyl-4-pyrimidine-carboxylique.

**4.** Utilisation du composé de formule Ia ou Ib selon l'une ou plusieurs des revendications 1 à 3, **caractérisée en ce que** l'on utilise la préparation cosmétique sous forme d'une solution, d'une suspension, d'une émulsion, d'une pâte, d'un onguent, d'un gel, d'une crème, d'une lotion, d'une poudre, d'un savon, d'un produit nettoyant contenant des agents tensio-actifs, d'une huile, d'un rouge à lèvres, d'un stick de soins pour lèvres, d'un mascara, d'un eyeliner, de fard, d'un maquillage sous forme de poudre, d'émulsion ou de cire, d'une préparation pour la protection solaire et d'une préparation après-soleil ou d'un spray.

**5.** Utilisation du composé de formule Ia ou Ib selon l'une ou plusieurs des revendications 1 à 4 pour la fabrication de préparations cosmétiques destinées aux soins et à la prévention d'une peau vieillie, sèche et/ou irritée et d'un cuir chevelu sec, squameux.

**6.** Préparation cosmétique contenant au moins un composé de formule Ia ou Ib

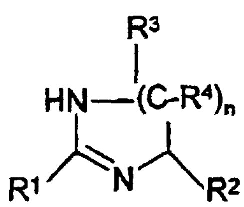

Ib

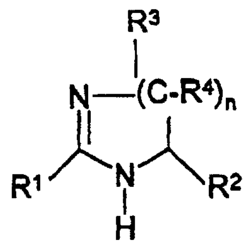

Ia

et/ou un sel physiologiquement compatible du composé de formule Ia ou Ib et/ou une forme stéréoisomère du composé de formule Ia ou Ib,

$R^1$ ayant la signification ci-après :

a) un atome d'hydrogène ou
b) un alkyle en $C_1$-$C_4$,

$R^2$ ayant la signification ci-après :

a) un atome d'hydrogène,
b) -COOH
c)

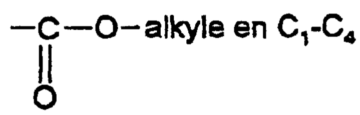

ou
d)

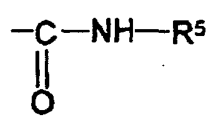

$R^5$ représentant

1) un atome d'hydrogène,
2) un alkyle en $C_1$-$C_4$,
3) un reste acide aminé,
4) un reste dipeptide, ou
5) un reste tripeptide,

$R^3$ et $R^4$ ayant, indépendamment l'un de l'autre, la signification d-après :

a) un atome d'hydrogène ou

b) -OH et

n est égal à 1, 2 ou 3,
**caractérisé en ce que** la préparation est une émulsion.